# EUROPÄISCHE PATENTSCHRIFT

(11) **EP 4 151 186 B1**
(45) Veröffentlichungstag und Bekanntmachung des Hinweises auf die Patenterteilung: **27.03.2024**
(21) Anmeldenummer: 22196209.5
(22) Anmeldetag: 16.09.2022
(51) Int. Cl.: A61F 9/007, A61M 39/10, A61M 39/12

(54) **KASSETTE FÜR EINE KONSOLE EINES OPHTHALMOCHIRURGISCHEN SYSTEMS SOWIE OPHTHALMOCHIRURGISCHES SYSTEM**
CASSETTE FOR A CONSOLE OF AN OPHTHALMOSURGICAL SYSTEM AND OPHTHALMOSURGICAL SYSTEM
CARTOUCHE POUR CONSOLE DE SYSTÈME DE CHIRURGIE OPHTALMOLOGIQUE ET SYSTÈME DE CHIRURGIE OPHTALMOLOGIQUE

(30) Priorität: 21.09.2021 DE 102021124413
(43) Veröffentlichungstag der Anmeldung: 22.03.2023
(73) Patentinhaber: Carl Zeiss Meditec AG, 07745 Jena (DE)
(72) Erfinder: Werner, Andreas, 73431 Aalen (DE); Kibbel, Steffen, 73466 Lauchheim (DE); Saltini, Giulio, 40017 San Giovanni in Persiceto (BO) (IT); Eichert, Peter, 86657 Bissingen (DE)
(74) Vertreter: Hofstetter, Schurack & Partner

(56) Entgegenhaltungen:
- EP-B1- 2 252 345
- EP-B1- 3 021 803
- WO-A1-94/22520
- DE-U1-202016 101 475
- GB-A- 2 451 891
- US-A1- 2006 157 984

## Beschreibung

Die Erfindung betrifft eine Kassette für eine Konsole eines ophthalmochirurgischen Systems zur Behandlung eines Auges, wobei die Kassette zum Einsetzen in einen Kassettenaufnahmebereich der Konsole ausgebildet ist und wenigstens eine Fluidpumpe zum Fördern eines Behandlungsfluids, einen Schlauch zum Anschließen an ein Handstück des ophthalmochirurgischen Systems sowie eine Verbindungseinheit zum gegen ein sich-Lösen gesicherten mechanischen Verbinden eines kassettenseitigen Endes des Schlauches mit einem Kassettengehäuse der Kassette aufweist. Ferner betrifft die Erfindung ein ophthalmochirurgisches System zur Behandlung eines Auges, mit zumindest einer Kassette, die wenigstens eine Fluidpumpe zum Fördern eines Behandlungsfluids aufweist, einer Konsole, die einen Kassettenaufnahmebereich zum Aufnehmen der Kassette und Antriebsmittel zum Antreiben der in der Kassette angeordneten Fluidpumpe aufweist, und einem ophthalmochirurgischen Handstück zum Behandeln einer Augenlinse des Auges, welches zumindest mit der Kassette fluidtechnisch koppelbar ist.

Ophthalmochirurgische Systeme, die Konsolen und hiermit koppelbare ophthalmochirurgische Handstücke aufweisen, sowie Kassetten für eine Konsole eines ophthalmochirurgischen Systems zur Behandlung eines Auges sind im Stand der Technik bekannt, sodass es diesbezüglich eines gesonderten druckschriftlichen Nachweises dem Grunde nach nicht bedarf. Das Handstück ist in der Regel mit der Konsole, insbesondere der in der Konsole eingesetzten Kassette, über einen Schlauch fluidtechnisch gekoppelt. Beispielsweise zur Behandlung einer Augenlinsentrübung, in der Medizin auch als grauer Star bezeichnet, sind unterschiedliche chirurgische Techniken bekannt. Am weitesten verbreitet ist die Phakoemulsifikation, bei der eine dünne Hohlnadel in einen Kapselsack, in dem die Augenlinse angeordnet ist, eingeführt und zu Ultraschallschwingungen angeregt wird. Mittels der vibrierenden Hohlnadel kann die Linse emulsifiziert werden, wobei hierbei freigesetzte Linsenpartikel über eine Aspirationsleitung mittels einer Pumpe abgesaugt werden können. Dabei wird ein Spülfluid, auch Irrigationsfluid genannt, zugeführt. Die Linsenpartikel werden zusammen mit dem Fluid als Aspirationsfluid abgesaugt. Sobald die Linse vollständig emulsifiziert und entfernt ist, kann in den dann geleerten Kapselsack eine neue künstliche Linse eingesetzt werden. Hierdurch kann für den behandelten Patienten wieder ein gutes Sehvermögen erreicht werden.

Ein fortschrittliches ophthalmochirurgisches System, welches sich für die Phakoemulsifikation als besonders geeignet herausgestellt hat, offenbart zum Beispiel die DE 10 2016 201 297 B3. Bei diesem System werden jeweils zwei strömungstechnisch parallelgeschaltete Fluidpumpen für die Irrigation sowie auch für die Aspiration genutzt. Jede der Fluidpumpen weist eine Pumpkammer und eine mittels eines elastischen Trennelements von der Pumpkammer getrennte Antriebskammer auf. Die Antriebskammer wird für den bestimmungsgemäßen Betrieb der Fluidpumpe mit einem Antriebsfluid beaufschlagt, dessen Antriebsdruck zur Ausführung eines jeweiligen Pumphubs variiert wird. Dadurch verändert sich abhängig hiervon eine Auslenkungsposition des elastischen Trennelements, die sich entsprechend auf die Pumpkammer auswirkt. Die Pumpkammer ist mit dem jeweiligen Behandlungsfluid, beispielsweise dem Irrigationsfluid, dem Aspirationsfluid oder dergleichen, beaufschlagt. Durch geeignetes Steuern eines Einlass- und eines Auslassventils der Fluidpumpe, kann dann die Förderwirkung erreicht werden.

Eine Auslenkungsposition des elastischen Trennelements wird mittels eines der jeweiligen Fluidpumpe zugeordneten Auslenkungspositionssensors erfasst. Eine Steuereinrichtung des ophthalmochirurgischen Systems, insbesondere der Konsole steuert die Funktion der Fluidpumpe zumindest abhängig von einem Sensorsignal des Auslenkungspositionssensors und einem mittels eines Antriebsdrucksensors bereitgestellten Antriebsdrucksignal. Die Steuereinrichtung kann ergänzend beispielsweise das Einlass- und das Auslassventil entsprechend steuern.

Durch wechselseitiges Betätigen der jeweils zwei parallelgeschalteten Fluidpumpen kann während einer Operation ein Volumenstrom mit sehr geringen Schwankungen erreicht werden. Dadurch kann sich ein nahezu konstanter Augeninnendruck im Kapselsack einstellen. Solange ausreichend Irrigationsfluid zugeführt werden kann, kann das System auch während einer sehr lang andauernden Operation nahezu ohne Unterbrechung des Irrigationsfluidstroms betrieben werden.

Für die Versorgung des Handstücks weist das ophthalmochirurgische System in der Regel wenigstens einen Schlauch auf, der die Konsole mit dem Handstück, insbesondere fluidtechnisch, koppelt, sodass dem Handstück einerseits das Irrigationsfluid zugeführt und andrerseits das Aspirationsfluid vom Handstück abgeführt werden kann. In der Regel sind zumindest die mit dem Behandlungsfluid in Kontakt stehenden Elemente in der separat ausgeführten Kassette angeordnet, die auswechselbar in einem Kassettenaufnahmebereich der Konsole einsetzbar ist. Üblicherweise ist daher der konsolenseitige Anschluss des wenigstens einen Schlauchs an der Kassette vorgesehen. Im Stand der Technik ist der Schlauch häufig mit der Kassette fest verbunden, indem er zum Beispiel mit dem Kassettengehäuse über eine Schweiß- oder Klebverbindung verbunden ist. Dadurch kann erreicht werden, dass die Verbindung des Schlauchs mit der Kassette gegen ein sich-Lösen gesichert werden kann. Hierdurch kann eine verbesserte Sterilität des ophthalmochirurgischen Systems erreicht werden. Zum Stand der Technik wird das Dokument EP 3021803 zitiert.

Diese starre Verbindung erweist sich jedoch dahingehend als nachteilig, als dass die Handhabung des Handstücks im bestimmungsgemäßen Betrieb des ophthalmochirurgischen Systems durch den, insbesondere unflexiblen, Ansatz an der Kassette erschwert sein kann. Darüber hinaus erweist es sich als nachteilig, dass der Schlauch bei einer Lagerung oder auch einem Transport der Kassette, beispielsweise in einer Verpackung, leicht geknickt werden kann, wodurch sich seine Eigenschaften ungünstig verändern können, besonders weil der Schlauch mit der Kassette fest verbunden ist.

Der Erfindung liegt die Aufgabe zugrunde, eine Kassette für den Einsatz in einer Konsole eines ophthalmochirurgischen Systems und ein solches System mit höherer Betriebssicherheit zu schaffen.

Als Lösung werden mit der Erfindung eine Kassette für eine Konsole eines ophthalmochirurgischen Systems zur Behandlung eines Auges sowie ein ophthalmochirurgisches System zur Behandlung des Auges gemäß den unabhängigen Ansprüchen vorgeschlagen.

Vorteilhafte Weiterbildungen ergeben sich durch Merkmale der abhängigen Ansprüche.

In Bezug auf eine gattungsgemäße Kassette für eine Konsole eines ophthalmochirurgischen Systems zur Behandlung eines Auges wird mit der Erfindung insbesondere vorgeschlagen, dass die Verbindungseinheit einen am Kassettengehäuse angeordneten Anschlussstutzen, der eine Durchgangsöffnung für das Behandlungsfluid aufweist, und einen am kassettenseitigen Ende des Schlauches ausgebildeten Steckverbinder aufweist, der ebenfalls eine Durchgangsöffnung für das Behandlungsfluid aufweist, wobei der Steckverbinder im mit dem Anschlussstutzen verbundenen Zustand gegenüber dem Anschlussstutzen fluiddicht und um eine Längsachse der Durchgangsöffnung drehbar gehalten ist.

In Bezug auf ein gattungsgemäßes ophthalmochirurgisches System wird mit der Erfindung insbesondere vorgeschlagen, dass die Kassette gemäß der Erfindung ausgebildet ist.

Die Erfindung basiert unter anderem auf dem Gedanken, dass durch eine gegen sich-Lösen gesicherte mechanische Verbindung, die eine Drehbarkeit um eine Längsachse der Durchgangsöffnung ermöglicht, die Flexibilität des Schlauches gegenüber der Kassette beziehungsweise der Konsole so verbessert werden kann, dass die eingangs beschriebenen Nachteile zumindest teilweise ausgeräumt werden können. Durch die Drehbarkeit des Schlauches gegenüber der Kassette ist es nämlich möglich, nicht nur das Risiko des Abknickens des Schlauches, insbesondere während einer bestimmungsgemäßen Nutzung des ophthalmochirurgischen Systems sowie innerhalb einer Transportverpackung, zu reduzieren, sondern es kann auch eine verbesserte Führung des Schlauches während einer Operation am Auge erreicht werden. Dies erleichtert nicht nur den Transport und die Lagerung, sondern auch die bestimmungsgemäße Nutzung und führt zu einer höheren Betriebssicherheit.

Durch die Verbindungseinheit der Erfindung kann erreicht werden, dass der Schlauch gegenüber der Kassette drehbar ist, obwohl er mit der Kassette derart verbunden ist, dass er gegen ein sich-Lösen gesichert ist. Dadurch kann einerseits eine Verbindung wie im Stand der Technik erreicht werden, die insbesondere aus Sicht der Sterilität dauerhaft besteht, sobald während der Herstellung die Kassette mit dem Schlauch verbunden worden ist, und andrerseits kann eine flexible Handhabung des Schlauchs erreicht werden, mit der die Nachteile in Bezug auf die bestimmungsgemäße Nutzung sowie Lagerung und Transport reduziert werden können. Die Verbindungseinheit ist daher ausgebildet, einmalig eine Verbindung herzustellen, die vorzugsweise nicht wieder lösbar ist, ohne die Kassette und/oder den Schlauch, insbesondere die Verbindungseinheit, zu beschädigen beziehungsweise zu zerstören.

Auch wenn vorliegend vorgesehen ist, dass das Kassettengehäuse den Anschlussstutzen und der Schlauch den Steckverbinder aufweist, kann im Rahmen einer kinematischen Umkehr natürlich auch eine duale Ausgestaltung vorgesehen sein, wobei der Steckverbinder an der Kassette und der Anschlussstutzen am Schlauch vorgesehen sind. Für eine derartige Verbindung gelten die gleichen Überlegungen.

Darüber hinaus erlaubt es die Erfindung, für den Schlauch und die Kassette, insbesondere das Kassettengehäuse, voneinander verschiedene Werkstoffe einzusetzen, und zwar unabhängig davon, ob diese Werkstoffe für ein gegenseitiges Verkleben beziehungsweise Verschweißen geeignet sind. Dadurch kann die Werkstoffauswahl für den Schlauch und das Kassettengehäuse individueller angepasst auf die jeweiligen Betriebs- und Nutzungserfordernisse erfolgen.

Sowohl der Anschlussstutzen als auch der Steckverbinder weisen jeweils eine Durchgangsöffnung für das Behandlungsfluid auf. Im verbundenen Zustand kann das Behandlungsfluid durch die Durchgangsöffnungen geführt werden. Zu diesem Zweck stehen die Durchgangsöffnungen im verbundenen Zustand miteinander fluidtechnisch in Verbindung.

Die Durchgangsöffnungen des Anschlussstutzens und des Steckverbinders können hinsichtlich eines Innenquerschnitts im Wesentlichen gleich ausgebildet sein. Je nach Konstruktion kann der Innenquerschnitt der Durchgangsöffnungen jedoch auch zumindest teilweise voneinander abweichen. Vorzugsweise ist jedoch eine Querschnittsfläche der jeweiligen Innenquerschnitte der Durchgangsöffnungen im Wesentlichen gleich. Dies gilt natürlich auch für eine Kontur eines Randes der jeweiligen Querschnittsflächen.

Durch in geeigneter Weise korrespondierend ausgebildete Anschlussstutzen und Steckverbinder kann zugleich auch eine im Wesentlichen fluiddichte Verbindung im zusammengefügten Zustand erreicht werden. Vorzugsweise ist die Verbindungseinheit derart ausgebildet, dass eine Drehbarkeit über einen Drehwinkel von mindestens etwa 120° erreicht werden kann. Vorzugsweise beträgt der Drehwinkel etwa 0° bis etwa 240°. Besonders vorteilhaft ist eine unbegrenzte Drehbewegung in Bezug auf den Winkel ermöglicht.

Der Steckverbinder weist eine Einstecköffnung für den Anschlussstutzen und der Anschlussstutzen einen Einsteckvorsprung zum Einstecken in die Einstecköffnung auf, wobei die jeweiligen Durchgangsöffnungen in die Einstecköffnung beziehungsweise den Einsteckvorsprung münden, sodass die Durchgangsöffnungen zueinander fluchten, wobei der Steckverbinder wenigstens ein Rastelement aufweist, das ausgebildet ist, mit einem korrespondierenden Rastelement des Anschlussstutzens zu verrasten. Dadurch kann die Verbindungseinheit auf einfache Weise geschaffen werden, indem der Steckverbinder mit dem Anschlussstutzen verbunden wird. Die jeweiligen Rastelemente verrasten gegenüber einander, sodass zumindest in axialer Richtung eine mechanisch feste Verbindung erreicht werden kann. Durch geeignete Ausgestaltung der Rastelemente kann die Drehbarkeit erreicht werden. Dabei kann vorgesehen sein, dass die Rastelemente zumindest teilweise von außen nicht zugänglich sind, sodass ein Entrasten ohne Beschädigen der Verbindungseinheit weitgehend unterbunden ist. Die Rastelemente können zu diesem Zweck in einem Bereich der Einstecköffnung und des Einsteckvorsprungs angeordnet sein. Nach der Verbindung kann auf diese Weise sichergestellt werden, dass die Verbindungseinheit nicht mehr zerstörungsfrei in den gelösten Zustand gebracht werden kann.

Darüber hinaus wird vorgeschlagen, dass das Rastelement des Anschlussstutzens zumindest als Rastnase ausgebildet ist, welche in eine, vorzugsweise umlaufende, Rastaufnahme des Steckverbinders einrastbar ist. Am Anschlussstutzen braucht jedoch nicht nur eine einzelne Rastnase vorgesehen zu sein. Je nach Bedarf kann auch eine Mehrzahl von Rastnasen ausgebildet sein, die beispielsweise verteilt über einen Umfang des Anschlussstutzens angeordnet sein können. Zumindest teilweise können die Rastnasen auch axial gegen einander versetzt sein. Die Rastaufnahme des Steckverbinders kann beispielsweise durch eine Nut oder auch durch Ausnehmungen gebildet sein. Vorzugsweise handelt es sich um eine umlaufende Nut, sodass eine unbegrenzte Drehbarkeit des Schlauches gegenüber dem Kassettengehäuse beziehungsweise der Kassette erreicht werden kann. Die Rastaufnahme ist vorzugsweise innenseitig in der Rastöffnung des Steckverbinders angeordnet, sodass sie von außen im Wesentlichen nicht zugänglich ist.

Ferner wird vorgeschlagen, dass die Verbindungseinheit ein Dichtungselement aufweist. Mittels des Dichtungselements kann die fluidtechnische Dichtung im Bereich der Verbindungseinheit verbessert werden. Insbesondere kann das Dichtungselement an das Behandlungsfluid angepasst ausgebildet sein, sodass neben einer guten Abdichtung auch eine, insbesondere chemische, Wechselwirkung mit dem Behandlungsfluid weitgehend vermieden werden kann. Zu diesem Zweck kann ein Werkstoff des Dichtungselements entsprechend gewählt sein. Darüber hinaus kann durch das Dichtungselement auch die Sterilität weiter verbessert werden, weil das Eindringen von Keimen von außerhalb der Verbindungseinheit weiter reduziert werden kann. Das Dichtungselement kann zum Beispiel als O-Ring, als Dichtungsscheibe, Kombinationen hiervon oder dergleichen gebildet sein. Im Grunde nach können natürlich auch mehrere unterschiedliche Dichtungselemente vorgesehen sein, die gemeinsam die Abdichtung der Verbindungseinheit bereitstellen oder verbessern. Ein weiterer Vorteil des Dichtungselements ist, dass altersbedingtes Kriechen oder Verformen der Werkstoffe der Verbindungseinheit ausgeglichen werden und somit über die gesamte Produktlebensdauer eine dichte Verbindung erreichbar ist.

Ferner wird vorgeschlagen, dass ein an den Steckverbinder angrenzender Abschnitt des Schlauchs bezogen auf die Längsachse abgewinkelt ist und insbesondere mit der Längsachse einen Winkel von etwa 90° bildet. Dadurch kann erreicht werden, dass zusammen mit der Drehbarkeit eine besonders günstige Positionierung des Schlauchs gegenüber dem Kassettengehäuse und somit insgesamt gegenüber der Kassette beziehungsweise der Konsole verbessert werden kann.

Die für die erfindungsgemäße Kassette angegebenen Vorteile und Wirkungen gelten natürlich gleichermaßen auch für das mit der erfindungsgemäßen Kassette ausgerüstete ophthalmochirurgisches System und umgekehrt.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Fig. nicht explizit gezeigt und erläutert sind, jedoch durch separierte Merkmalskombinationen aus den erläuterten Ausführungen hervorgehen und erzeugbar sind.

In den Figuren zeigen:
- Fig. 1: eine schematische Blockdarstellung eines erfindungsgemäßen ophthalmochirurgischen Systems mit einer Konsole und einem Handstück, welches mittels eines Schlauchs einer in der Konsole eingesetzten Kassette gekoppelt ist,
- Fig. 2: eine schematische Blockdarstellung einer ersten Ausführungsform des erfindungsgemäßen ophthalmochirurgischen Systems,
- Fig. 3: eine schematisch perspektivische Darstellung einer Konsole des Systems gemäß Fig. 2,
- Fig. 4: eine schematische Draufsicht auf eine Anschlussseite einer Kassette für das System gemäß Fig. 2,
- Fig. 5: eine schematisch perspektivische Ansicht der Kassette gemäß Fig. 4 auf eine Bedienseite, die der Anschlussseite gegenüberliegt, und
- Fig. 6: eine schematische Schnittdarstellung eines Ausschnitts der Kassette gemäß Fig. 5 im Bereich einer Verbindung mit einem Schlauch für eines der Behandlungsfluide.

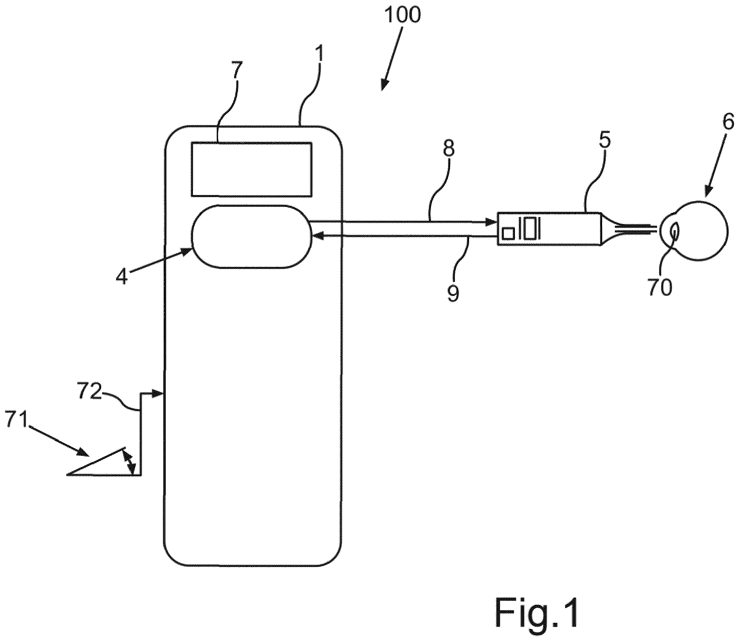

Fig. 1 zeigt in einer schematischen Blockdarstellung ein ophthalmochirurgisches System 100, welches zur Behandlung eines Auges 6 dient. Das ophthalmochirurgische System 100 weist ein chirurgisches Instrument 5 als Handstück auf. Ferner weist das ophthalmochirurgische System 100 eine Konsole 1 zum Anschließen und Betreiben des Handstücks 5 in einem bestimmungsgemäßen Betrieb auf. Weiterhin weist das ophthalmochirurgische System 100 eine Fußbedieneinheit 71 auf, die vorliegend als Fußpedal ausgebildet ist. Die Fußbedieneinheit 71 ist drahtlos mit der Konsole 1 koppelbar. Ferner weist die Konsole 1 eine Steuereinheit 7 sowie fluidische Versorgungseinheiten auf, die unter anderem dazu dienen, dem Handstück 5 über einen Irrigationsfluid-Strömungspfad 8 ein Irrigationsfluid 3 zuzuführen und über einen Aspirationsfluid-Strömungspfad 9 ein Aspirationsfluid vom Handstück 5 abzuführen. Vorliegend sind der Irrigationsfluid-Strömungspfad 8 und der Aspirationsfluid-Strömungspfad 9 jeweils durch einen Schlauch 61 bereitgestellt.

Die Fußbedieneinheit 71 ist vorliegend kabellos ausgebildet, weshalb sie zumindest während des bestimmungsgemäßen Betriebs von einem nicht weiter dargestellten elektrischen Energiespeicher mit elektrischer Energie versorgt wird. Darüber hinaus ist die Fußbedieneinheit 71 im bestimmungsgemäßen Betrieb über eine drahtlose Kommunikationsverbindung 72 mit der Steuereinheit 7 in Kommunikation, sodass ein Betriebszustand des Handstücks 5 zumindest teilweise eingestellt werden kann.

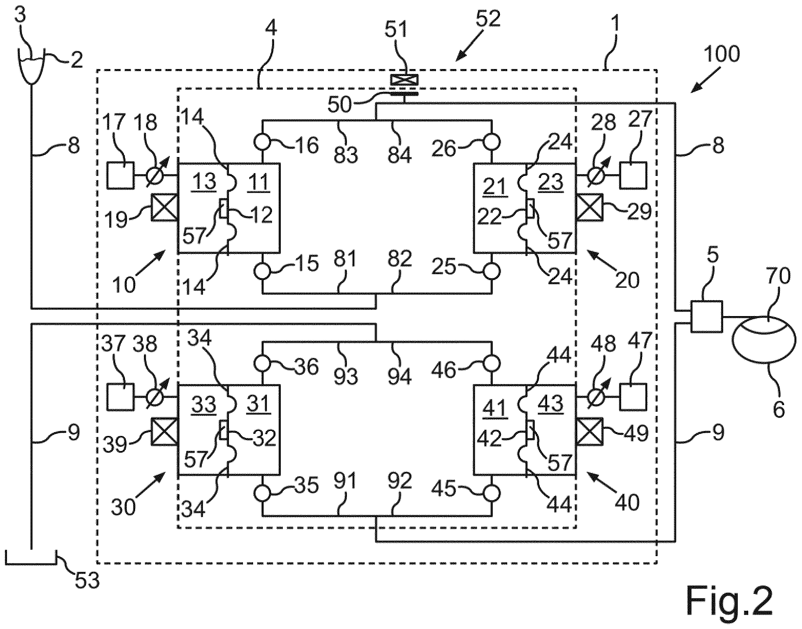

Fig. 2 zeigt eine schematische Blockdarstellung des ophthalmochirurgischen Systems 100 gemäß Fig. 1. Das System 100 weist die Konsole 1 auf, an welcher ein Irrigationsfluidbehälter 2 mit einem darin enthaltenen Irrigationsfluid 3 gekoppelt ist.

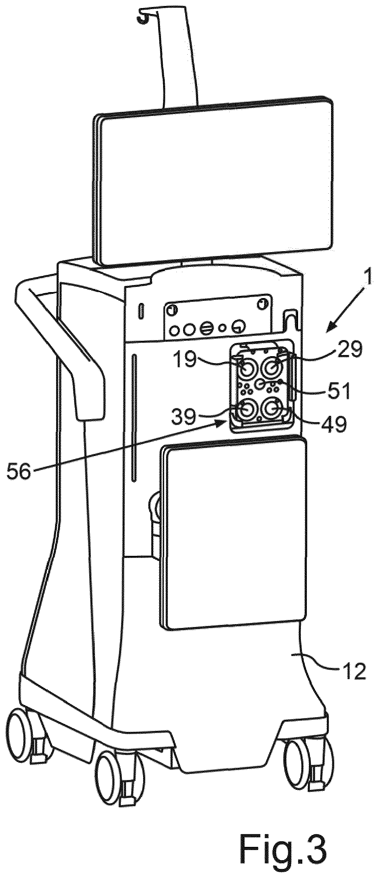

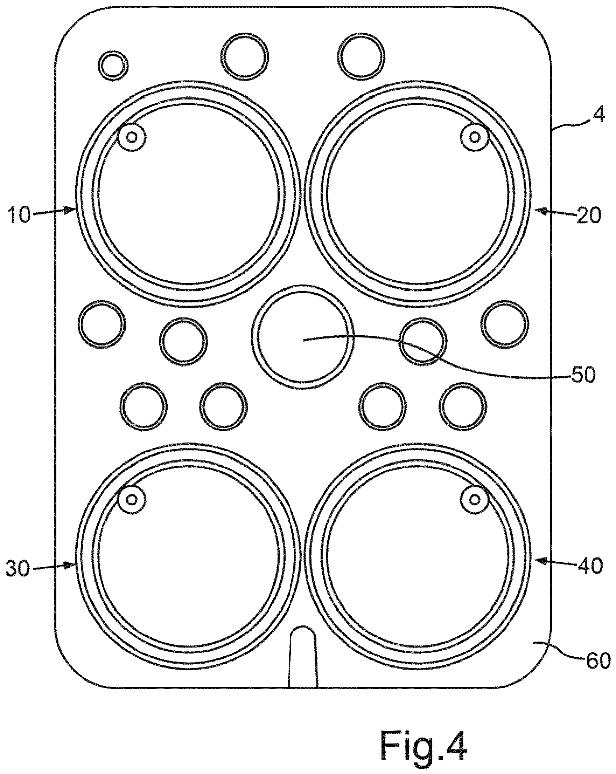

Weiterhin weist das System 100 eine Kassette 4 auf, welche in den Kassettenaufnahmebereich 56 der Konsole 1 einsetzbar ist (vergleiche Fig. 3). Die Kassette 4 dient neben einer Förderung des Irrigationsfluids 3 zum Handstück 5, welches zur Phakoemulsifikation einer Linse 70 des Auges 6 dient, auch zum Abführen eines hierbei anfallenden Aspirationsfluids aus einem Behandlungsbereich des Auges 6. Das Handstück 5 dient vorliegend zur Phakoemulsifikation der Linse 70 des Auges 6. Fig. 3 zeigt in einer schematischen perspektivischen Darstellung die Konsole 1 ohne die Kassette 4. Fig. 4 zeigt in einer schematischen Draufsicht eine Anschlussseite der Kassette 4 zum Verbinden mit der Konsole 1 im Kassettenaufnahmebereich 56.

Das System 100 weist ferner den Irrigationsfluid-Strömungspfad 8 auf, der sich vom Irrigationsfluidbehälter 2 über die Kassette 4 zum Handstück 5 erstreckt. Darüber hinaus weist das System 100 eine erste Fluidpumpe 10 mit einer ersten Pumpkammer 11 und eine mit einem ersten Trennelement 12 davon getrennten ersten Antriebskammer 13 auf. Das erste Trennelement 12 ist bevorzugt verformbar ausgebildet. Das erste Trennelement 12 weist einen Rand 14 auf, mittels dem es mit der Fluidpumpe 10 verbunden ist. Das Trennelement 12 ist an seinem Rand 14 in einer axialen Richtung, welche parallel zu einer Trennelement-Mittelachse verläuft, nicht verlagerbar. Bevorzugt ist das Trennelement 12 an seinem Rand 14 fest eingespannt.

Das Irrigationsfluid 3 ist über den Irrigationsfluid-Strömungspfad 8 und einem ersten Einlassventil 15 der ersten Pumpkammer 11 abhängig von einem Ventilzustand des Einlassventils 15 der ersten Pumpkammer 11 zuführbar. Ferner ist es über ein Auslassventil 16 abhängig von dessen Ventilzustand wieder von der Pumpkammer 11 abführbar. Die erste Antriebskammer 13 kann mit einem ersten Antriebsfluid 17 beaufschlagt werden, welches mittels eines in der Konsole 1 angeordneten Proportionalventils 18 zugeführt werden kann. Abhängig von einem Differenzdruck zwischen dem ersten Antriebsfluid 17 in der ersten Antriebskammer 13 und dem Irrigationsfluid als Behandlungsfluid in der ersten Pumpkammer 11 kommt es zu einer elastischen Verformung beziehungsweise Auslenkung oder Verlagerung des ersten Trennelements 12. Dabei ist ein Betrag des Drucks in der ersten Antriebskammer 13 größer als ein Betrag des Drucks in der ersten Pumpkammer 11. Wenn das Einlassventil 15 geschlossen und das Auslassventil 16 geöffnet ist, kann das Irrigationsfluid aus der ersten Pumpkammer 11 in einen an das Auslassventil angeschlossenen Teilpfad 83 ausströmen.

Eine Auslenkungsposition des ersten Trennelements 12 kann mittels eines ersten Auslenkungspositionssensors 19 erfasst werden, welcher außerhalb der ersten Fluidpumpe 10 zum Beispiel in der Konsole 1 angeordnet ist. Der erste Auslenkungspositionssensor 19 ist vorliegend als Sensoreinheit nach Art eines induktiven Wegsensors ausgebildet. Die Funktion des Auslenkungspositionssensors 19 wird im Folgenden noch erläutert werden.

Wie aus Fig. 1 ersichtlich ist, sind die Antriebskammer 13 und die Pumpkammer 11 mit dem Trennelement 12 in der Kassette 4 angeordnet. Durch Anordnen der Kassette 4 in der Konsole 1 wird die Fluidpumpe 10 mit einer Antriebsfluidzuführung der Konsole 1 und dem in der Konsole 1 angeordneten Auslenkungspositionssensor 19 gekoppelt, sodass die gewünschte Pumpfunktion und die Erfassungsfunktion für das Erfassen der Auslenkungsposition des ersten elastischen Trennelements 12 erreicht werden kann.

Aus Fig. 2 ist ferner ersichtlich, dass zur ersten Fluidpumpe 10 eine zweite Fluidpumpe 20 strömungstechnisch parallelgeschaltet ist. Die zweite Fluidpumpe 20 ist vorliegend wie die erste Fluidpumpe 10 ausgebildet. Daher teilt sich der Irrigationsfluid-Strömungspfad 8 in der Kassette 4 in einen ersten Teilpfad 81 und einen zweiten Teilpfad 82 auf. Der erste Teilpfad 81 ist an das erste Einlassventil 15 und der zweite Teilpfad 82 an ein zweites Einlassventil 25 der zweiten Fluidpumpe 20 angeschlossen.

Die zweite Fluidpumpe 20 weist eine zweite Pumpkammer 21 und eine hiervon mittels eines zweiten Trennelements 22 getrennte zweite Antriebskammer 23 auf. Das Trennelement 22 weist einen zweiten Rand 24 auf, der zum Beispiel fest in der zweiten Fluidpumpe 20 montiert ist. Die zweite Antriebskammer 23 kann mit einem zweiten Antriebsfluid 27 über ein in der Konsole 1 angeordnetes zweites Proportionalventil 28 beaufschlagt werden. Eine Auslenkungsposition des Trennelements 22 kann mittels eines zweiten Auslenkungspositionssensors 29 erfasst werden, der vorliegend entsprechend dem ersten Auslenkungspositionssensor 19 ausgebildet ist. Über ein zweites Auslassventil 26 kann das Irrigationsfluid 3 die zweite Pumpkammer 21 in den Teilpfad 84 wieder verlassen. Über die Teilpfade 83, 84, die am jeweiligen ersten beziehungsweise zweiten Auslassventil 16, 26 angeschlossen sind, kann das die jeweilige Fluidpumpe 10, 20 verlassende Irrigationsfluid wieder dem Irrigationsfluid-Strömungspfad 8 zugeführt werden, um dem Instrument 5 zugeführt zu werden.

In einem Bereich der strömungstechnischen Verbindung des Teilpfades 83 mit dem Teilpfad 84, also zum Beispiel in dem nachfolgenden Irrigationsfluid-Strömungspfad 8, ist eine elastische Membran 50 ausgebildet, die das Irrigationsfluid kontaktieren kann. Die Membran 50 ist an der Kassette 4 angeordnet. Die Membran 50 wird von einem Kraftsensor 51 kontaktiert, der seinerseits in der Konsole 1 angeordnet ist, wenn die Kassette 4 im Kassettenaufnahmebereich 56 angeordnet ist (Fig. 4). Die Membran 50 bildet in Verbindung mit dem Kraftsensor 51 einen Erfassungssensor 52.

Während des Zerkleinerns der Augenlinse 70 werden kleine Linsenpartikel freigesetzt, die zusammen mit dem zugeführten Irrigationsfluid abgesaugt werden können. Das mit Linsenpartikeln verunreinigte Irrigationsfluid wird dann als Aspirationsfluid bezeichnet und über einen Aspirationsfluid-Strömungspfad 9 zu einem Aspirationsfluid-Sammelbehälter 53 gefördert. Zu diesem Zweck sind vorliegend zwei weitere parallelgeschaltete Fluidpumpen 30, 40 vorgesehen, die dem Grunde nach vergleichbar zu den Fluidpumpen 10, 20 für das Irrigationsfluid ausgebildet sind. Zu diesem Zweck ist innerhalb der Kassette 4 vorgesehen, dass der Aspirations-Strömungspfad 9 sich ebenfalls in zwei Teilpfade 91, 92 aufteilt, die über jeweilige Einlassventile 35, 45 an die jeweiligen Fluidpumpen 30, 40 angeschlossen sind, und zwar hier zu den jeweiligen Pumpkammern 31, 41. Auch hier sind die Pumpkammern 31, 41 über jeweilige Trennelemente 32, 42 von jeweiligen Antriebskammern 33, 43 getrennt. Die Trennelemente 32, 42 weisen jeweilige Ränder 34, 44 auf, die fest in der jeweiligen Fluidpumpe 30, 40 montiert sind. Über jeweilige Auslassventile 36, 46 und daran angeschlossene Teilpfade 93, 94 kann das Aspirationsfluid dann über den Aspirationsfluid-Strömungspfad 9 abgeführt werden. Ein drittes Antriebsfluid 37 kann mittels eines dritten Proportionalventils 38 zur dritten Antriebskammer 33 geführt werden. Entsprechend kann ein viertes Antriebsfluid 47 mittels eines vierten Proportionalventils 48 zu einer vierten Antriebskammer 43 geführt werden. Die Proportionalventile 38, 48 sind in der Konsole 1 angeordnet. Die Auslenkungspositionen der Trennelemente 32, 42 können mittels jeweiliger Auslenkungspositionssensoren 39, 49 erfasst werden. Die beiden Fluidpumpen 30, 40 werden vorliegend ebenfalls wechselweise wie die Fluidpumpen 10, 20 betrieben.

Fig. 3 zeigt in einer schematisch perspektivischen Darstellung die Konsole 1 des ophthalmochirurgischen Systems 100 gemäß Fig. 1, wobei die Darstellung eine Vorderseite der Konsole 1 umfasst. Aus Fig. 3 ist ersichtlich, dass die Konsole 1 den Kassettenaufnahmebereich 56 aufweist, der dem lösbaren Anordnen der Kassette 4 dient. Die Konsole 1 weist ferner ein nicht dargestelltes Antriebsfluidversorgungssystem auf, welches dazu dient, das jeweilige Antriebsfluid für die jeweiligen Antriebskammern 13, 23, 33, 43 bereitzustellen, welches vorliegend durch Luft gebildet ist. Das Antriebsfluidversorgungssystem weist für jede der Fluidpumpen 10, 20, 30, 40 jeweilige Quellen für Antriebsfluid 17, 27, 37, 47 (Fig. 1) auf, die an jeweilige Proportionalventile 18, 28, 38, 48 angeschlossen sind. Über die Proportionalventile 18, 28, 38, 48 kann die jeweilige Antriebskammer 13, 23, 33, 43 im bestimmungsgemäßen Betrieb mit dem jeweiligen Antriebsfluid beaufschlagt werden. Die Konsole 4 weist ein Konsolengehäuse 12 auf, in dem die Elemente beziehungsweise Einheiten der Konsole 4 angeordnet sind. Das Konsolengehäuse 12 stellt den Kassettenaufnahmebereich 56 bereit, in dem die Kassette 4 angeordnet werden kann.

Aus Fig. 2 ist ferner ersichtlich, dass jedes der elastischen Trennelemente 12, 22, 32, 42 ein Plattenelement 57 aufweist, welches beim Bewegen des elastischen Trennelements 12, 22, 32, 42 mitbewegt wird. Das Plattenelement 57 ist vorliegend als Metallplättchen ausgebildet und aus einem ferromagnetischen Werkstoff gebildet. Das Plattenelement 57 weist eine Dicke auf, die kleiner als eine Dicke des elastischen Trennelements 12, 22, 32, 42 ist.

Die Konsole 1 weist ferner für jede der Fluidpumpen 10, 20, 30, 40 einen jeweiligen Auslenkungspositionssensor 19, 29, 39, 49 auf, der vorliegend jeweils eine Sensoreinheit bereitstellt, die ein magnetisches Feld nutzt, um die Position des jeweiligen Plattenelements 57 und damit des jeweiligen der elastischen Trennelemente 12, 22, 32, 42 zu erfassen.

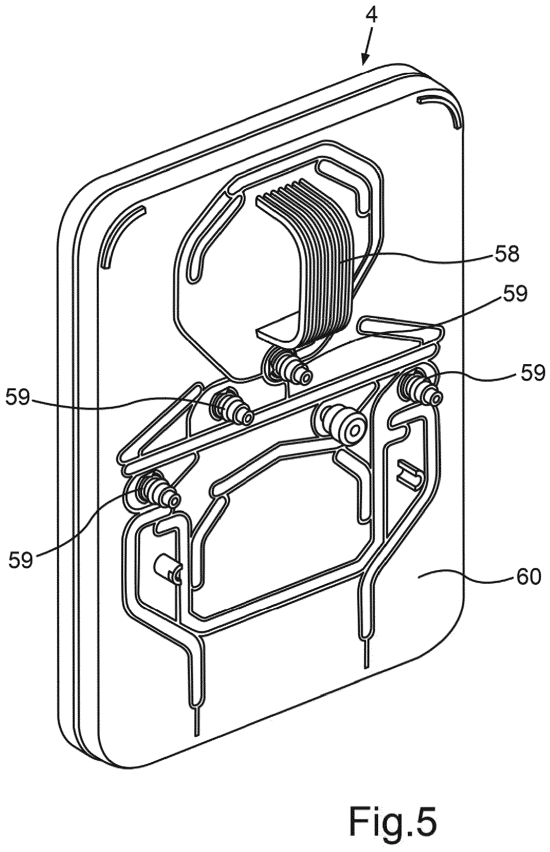

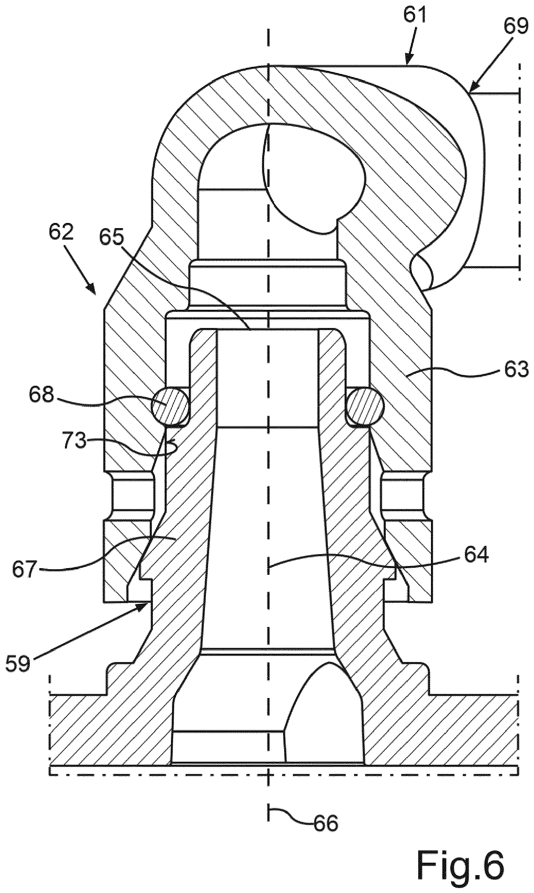

Fig. 4 zeigt in einer schematischen Draufsicht die Kassette 4 mit einer Anschlussseite, welche im im Kassettenaufnahmebereich 56 angeordneten Zustand der Konsole 1 zugewandt ist. Die Kassette 4 weist ein Kassettengehäuse 60 auf. Fig. 5 zeigt in einer perspektivischen Ansicht der Kassette 4 eine Bedienseite, die der Anschlussseite gegenüberliegt. Zu erkennen ist, dass die Kassette 4 in einem oberen Bereich eine Handhabe 58 aufweist, mittels der die Kassette 4 manuell in den Kassettenaufnahmebereich 56 einsetzbar beziehungsweise aus diesem entnehmbar ist. Darüber hinaus sind an der Bedienseite Anschlussstutzen 59 vorgesehen, die, wie im Folgenden noch erläutert werden wird, jeweils mit einem Schlauch 61 mechanisch verbunden sind, und zwar derart, dass die hierdurch geschaffene Verbindung gegen ein sich-Lösen gesichert ist. Zu diesem Zweck ist eine Verbindungseinheit 62 (Fig. 6) vorgesehen. In Fig. 5 sind lediglich die Anschlussstutzen 59 der Verbindungseinheit 62 ohne den Schlauch 61 sichtbar, das heißt, vor einer Verbindung des Kassettengehäuses 60 mit dem jeweiligen Schlauch 61.

Fig. 6 zeigt eine schematische Schnittansicht im Bereich einer der Verbindungseinheiten 62 der Kassette 4. Die Verbindungseinheit 62 weist den am Kassettengehäuse 60 angeordneten Anschlussstutzen 59 auf, der eine Durchgangsöffnung 64 für das Behandlungsfluid aufweist. Die Verbindungseinheit 62 weist ferner einen am kassettenseitigen Ende des Schlauches 61 ausgebildeten Steckverbinder 63 auf, der ebenfalls eine Durchgangsöffnung 65 für das Behandlungsfluid aufweist. Der Steckverbinder 63 ist in mit dem Anschlussstutzen 59 verbundenen Zustand gegenüber dem Anschlussstutzen 59 fluiddicht und um eine Längsachse 66 der Durchgangsöffnungen 64, 65 drehbar gehalten, wie dies in der schematischen Schnittansicht gemäß Fig. 6 dargestellt ist.

Der Steckverbinder 63 weist eine Einstecköffnung 73 für den Anschlussstutzen 59 und der Anschlussstutzen 59 einen Einsteckvorsprung 67 zum Einstecken in die Einstecköffnung 73 auf. Die jeweiligen Durchgangsöffnungen 64, 65 münden in die Einstecköffnung 73 beziehungsweise den Einsteckvorsprung 67, sodass die Durchgangsöffnungen 64, 65 im verbundenen Zustand zueinander fluchten. Der Steckverbinder 63 weist ein nicht dargestelltes Rastelement auf, welches ausgebildet ist, mit einem korrespondierenden Rastelement des Anschlussstutzens 59 zu verrasten. Das korrespondierende Rastelement des Anschlussstutzens 59 ist vorliegend durch mehrere Rastnasen ausgebildet, die in eine umlaufende Nut des Steckverbinders 63 einrastbar sind, die eine Rastaufnahme als Rastelement des Steckverbinder 63 bereitstellt. Die Rastnasen und die Nut sind in einem Verbindungsbereich der Verbindungseinheit 62 angeordnet, sodass sie zum Lösen der Rastverbindung nicht zugänglich sind. Dadurch ist die Verbindungseinheit 62 gegen ein sich-Lösen gesichert.

Zu erkennen ist ferner, dass die Verbindungseinheit 62 ein Dichtungselement 68 aufweist, welches zwischen einem stirnseitigen Rücksprung des Anschlussstutzens 59 und einer Innenseite der Einstecköffnung 73 angeordnet ist. Das Dichtungselement 68 ist vorliegend nach Art eines O-Rings ausgebildet. Es ist vorliegend elastisch ausgebildet, sodass eine gute Abdichtung trotz der Drehbarkeit erreicht werden kann.

Ein an den Steckverbinder 63 angrenzender Abschnitt 69 des Schlauchs 61 ist bezogen auf die Längsachse 66 abgewinkelt und bildet mit der Längsachse 66 einen Winkel von etwa 90°. Dadurch kann eine gut einstellbare Schlauchführung für den Schlauch 61 erreicht werden. Vorliegend ist vorgesehen, dass die hier dargestellte Verbindungseinheit 62 für sämtliche vier Anschlüsse der Kassette 4 entsprechend vorgesehen ist. Über die Schläuche 61 kann der Kassette 4 sowohl vom Reservoir 2 das Irrigationsfluid 3 zugeführt werden, sodass es über einen weiteren der Anschlüsse dem Handstück 5 zugeführt werden kann. Entsprechend kann der Kassette 4 über einen Schlauch 61 vom Handstück 5 das Aspirationsfluid zugeführt und über einen weiteren Anschlussstutzen 59 der Kassette 4 einem Aspirationsfluid-Sammelbehälter 53 für das Aspirationsfluid zugeführt werden.

Die Verbindungseinheit 62 wird bei der Herstellung der Kassette 4 vervollständigt, wobei in einem der letzten Herstellungsschritte die jeweiligen Steckverbinder 63 der Schläuche 61 zum Ausbilden der jeweiligen Verbindungseinheiten 62 auf die jeweiligen Anschlussstutzen 59 aufgesteckt und verrastet werden.

Die Ausführungsbeispiele dienen ausschließlich der Erläuterung der Erfindung und sollen diese nicht beschränken.

### Bezugszeichenliste

- 1: Konsole
- 2: Irrigationsfluidbehälter
- 3: Irrigationsfluid
- 4: Kassette
- 5: Handstück, chirurgisches Instrument
- 6: Auge
- 7: Steuereinheit
- 8: Irrigationsfluid-Strömungspfad
- 9: Aspirations-Strömungspfad
- 10: erste Fluidpumpe
- 11: erste Pumpkammer
- 12: erstes Trennelement
- 13: erste Antriebskammer
- 14: Rand
- 15: erstes Einlassventil
- 16: erstes Auslassventil
- 17: erstes Antriebsfluid
- 18: erstes Proportionalventil
- 19: Auslenkungspositionssensor
- 20: zweite Fluidpumpe
- 21: zweite Pumpkammer
- 22: zweites Trennelement
- 23: zweite Antriebskammer
- 24: Rand
- 25: zweites Einlassventil
- 26: zweites Auslassventil
- 27: zweites Antriebsfluid
- 28: zweites Proportionalventil
- 29: Auslenkungspositionssensor
- 30: dritte Fluidpumpe
- 31: dritte Pumpkammer
- 32: drittes Trennelement
- 33: dritte Antriebskammer
- 34: Rand
- 35: Einlassventil
- 36: Auslassventil
- 37: drittes Antriebsfluid
- 38: Proportionalventils
- 39: Auslenkungspositionssensor
- 41: vierte Pumpkammer
- 42: viertes Trennelement
- 43: vierte Antriebskammer
- 44: Rand
- 45: viertes Einlassventil
- 46: viertes Auslassventil
- 47: viertes Antriebsfluid
- 48: viertes Proportionalventil
- 49: Auslenkungspositionssensor
- 50: Membran
- 51: Kraftsensor
- 52: Erfassungssensor
- 53: Aspirationsfluid-Sammelbehälter
- 54: Verstelleinrichtung
- 55: Kopplung
- 56: Kassettenaufnahmebereich
- 57: Plattenelement
- 58: Handhabe
- 59: Anschlussstutzen
- 60: Kassettengehäuse
- 61: Schlauch
- 62: Verbindungseinheit
- 63: Steckverbinder
- 64: Durchgangsöffnung
- 65: Durchgangsöffnung
- 66: Längsachse
- 67: Einsteckvorsprung
- 68: Dichtungselement
- 69: Abschnitt
- 70: Linse
- 71: Fußbedieneinheit
- 72: Kommunikationsverbindung
- 73: Einstecköffnung
- 81, 82, 83, 84, 91, 92, 93, 94: Teilpfad
- 100: ophthalmochirurgisches System

## Patentansprüche

1. Kassette (4) für eine Konsole (1) eines ophthalmochirurgischen Systems (100) zur Behandlung eines Auges (6), wobei die Kassette (4) zum Einsetzen in einen Kassettenaufnahmebereich (56) der Konsole (1) ausgebildet ist und wenigstens eine Fluidpumpe (10, 20, 30, 40) zum Fördern eines Behandlungsfluids, einen Schlauch (61) zum Anschließen an ein Handstück (5) des ophthalmochirurgischen Systems (100) sowie eine Verbindungseinheit (62) zum gegen ein sich-Lösen gesicherten mechanischen Verbinden eines kassettenseitigen Endes des Schlauches (61) mit einem Kassettengehäuse (60) der Kassette (4) aufweist, **dadurch gekennzeichnet, dass**
die Verbindungseinheit (62) einen am Kassettengehäuse (60) angeordneten Anschlussstutzen (59), der eine Durchgangsöffnung (64) für das Behandlungsfluid aufweist, und einen am kassettenseitigen Ende des Schlauches (61) ausgebildeten Steckverbinder (63) aufweist, der ebenfalls eine Durchgangsöffnung (65) für das Behandlungsfluid aufweist, wobei der Steckverbinder (63) im mit dem Anschlussstutzen (59) verbundenen Zustand gegenüber dem Anschlussstutzen (59) fluiddicht und um eine Längsachse (66) der Durchgangsöffnung (64, 65) drehbar gehalten ist, wobei der Steckverbinder (63) eine Einstecköffnung (73) für den Anschlussstutzen (59) und der Anschlussstutzen (59) einen Einsteckvorsprung (67) zum Einstecken in die Einstecköffnung (73) aufweist wobei die jeweiligen Durchgangsöffnungen (64, 65) in die Einstecköffnung (73) beziehungsweise den Einsteckvorsprung (67) münden, sodass die Durchgangsöffnungen (64, 65) zueinander fluchten, wobei der Steckverbinder (63) wenigstens ein Rastelement aufweist, das ausgebildet ist, mit einem korrespondierenden Rastelement des Anschlussstutzens (59) zu verrasten.

2. Kassette nach Anspruch 1, wobei das Rastelement des Anschlussstutzens (59) zumindest als Rastnase ausgebildet ist, welche in eine, vorzugsweise umlaufende, Rastaufnahme des Steckverbinders (63) einrastbar ist.

3. Kassette nach einem der vorhergehenden Ansprüche, wobei die Verbindungseinheit ein Dichtungselement (68) aufweist.

4. Kassette nach einem der vorhergehenden Ansprüche, wobei ein an den Steckverbinder (63) angrenzender Abschnitt (69) des Schlauchs (61) bezogen auf die Längsachse (66) abgewinkelt ist und insbesondere mit der Längsachse (66) einen Winkel von 90° bildet.

5. Ophthalmochirurgisches System (100) zur Behandlung eines Auges (6), mit zumindest:
- einer Kassette (4), die wenigstens eine Fluidpumpe (10, 20, 30, 40) zum Fördern eines Behandlungsfluids aufweist,
- einer Konsole (1), die den Kassettenaufnahmebereich (56) zum Aufnehmen der Kassette (4) und Antriebsmittel zum Antreiben der in der Kassette angeordneten Fluidpumpe (10, 20, 30, 40) aufweist, und
- einem ophthalmochirurgischen Handstück (5) zum Behandeln einer Augenlinse (7) des Auges (6), welches zumindest mit der Kassette (4) fluidtechnisch koppelbar ist, wobei die Kassette (4) nach einem der vorhergehenden Ansprüche ausgebildet ist.

## Claims

1. Cassette (4) for a console (1) of an ophthalmosurgical system (100) for treating an eye (6), the cassette (4) being designed for insertion into a cassette receiving region (56) of the console (1) and having at least one fluid pump (10, 20, 30, 40) for delivering a treatment fluid, a tube (61) for attachment to a handpiece (5) of the ophthalmosurgical system (100), and a connection unit (62) for the mechanical connection, secured against detachment, of a cassette-side end of the tube (61) to a cassette housing (60) of the cassette (4), **characterized in that**
the connection unit (62) comprises an attachment stub (59), which is arranged on the cassette housing (60) and has a through opening (64) for the treatment fluid, and a plug-in connector (63), which is on the cassette-side end of the tube (61) and likewise has a through opening (65) for the treatment fluid, with the plug-in connector (63), when it is connected to the attachment stub (59), being held in a fluid-tight manner with respect to the attachment stub (59) and so as to be able to rotate about a longitudinal axis (66) of the through opening (64, 65), with the plug-in connector (63) having a plug-in opening (73) for the attachment stub (59) and the attachment stub (59) having a plug-in projection (67) for plugging into the plug-in opening (73), with the respective through openings (64, 65) leading into the plug-in opening (73) or the plug-in projection (67), respectively, with the result that the through openings (64, 65) are in line with one another, with the plug-in connector (63) having at least one latching element which is designed to latch to a corresponding latching element of the attachment stub (59).

2. Cassette according to Claim 1,
wherein
the latching element of the attachment stub (59) is at least in the form of a latching lug, which can be latched into a preferably encircling latching receptacle of the plug-in connector (63).

3. Cassette according to either one of the preceding claims,
wherein
the connection unit has a sealing element (68).

4. Cassette according to any one of the preceding claims,
wherein
a portion (69), adjoining the plug-in connector (63), of the tube (61) is angled away with respect to the longitudinal axis (66) and in particular forms an angle of 90° with the longitudinal axis (66).

5. Ophthalmosurgical system (100) for treating an eye (6), at least comprising:
- a cassette (4), which has at least one fluid pump (10, 20, 30, 40) for delivering a treatment fluid,
- a console (1), which has the cassette receiving region (56) for receiving the cassette (4) and drive means for driving the fluid pump (10, 20, 30, 40) that is arranged in the cassette, and
- an ophthalmosurgical handpiece (5) for treating a crystalline lens (7) of the eye (6), which handpiece is fluidically couplable at least to the cassette (4), wherein
the cassette (4) is designed according to any one of the preceding claims.

## Revendications

1. Cartouche (4) pour une console (1) d'un système de chirurgie ophtalmologique (100) destiné au traitement d'un œil (6), dans laquelle la cartouche (4) est réalisée pour être insérée dans une zone de réception de cartouche (56) de la console (1) et présente au moins une pompe à fluide (10, 20, 30, 40) pour débiter un fluide de traitement, un tuyau (61) destiné à être raccordé à une pièce à main (5) du système de chirurgie ophtalmologique (100), ainsi qu'une unité de raccordement (62) pour un raccordement mécanique, protégé contre le détachement, d'une extrémité côté cartouche du tuyau (61) à un boîtier de cartouche (60) de la cartouche (4),
**caractérisée en ce que** l'unité de raccordement (62) présente un embout de raccordement (59) disposé sur le boîtier de cartouche (60) et qui présente une ouverture de passage (64) pour le fluide de traitement, et un connecteur enfichable (63) réalisé à une extrémité côté cartouche du tuyau (61) et qui présente également une ouverture de passage (65) pour le fluide de traitement, dans laquelle, à l'état raccordé à l'embout de raccordement (59), le connecteur enfichable (63) est maintenu de manière étanche aux fluides par rapport à l'embout de raccordement (59) et de manière à pouvoir tourner autour d'un axe longitudinal (66) de l'ouverture de passage (64, 65), dans laquelle le connecteur enfichable (63) présente une ouverture d'insertion (73) pour l'embout de raccordement (59), et l'embout de raccordement (59) présente une saillie d'insertion (67) pour l'insertion dans l'ouverture d'insertion (73), dans laquelle les ouvertures de passage (64, 65) respectives débouchent sur l'ouverture d'insertion (73) ou la saillie d'insertion (67) respectivement de sorte que les ouvertures de passage (64, 65) sont alignées l'une sur l'autre, dans laquelle le connecteur enfichable (63) présente au moins un élément d'enclenchement qui est réalisé pour s'enclencher dans un élément d'enclenchement correspondant de l'embout de raccordement (59).

2. Cartouche selon la revendication 1, dans laquelle l'élément d'enclenchement de l'embout de raccordement (59) est réalisé au moins sous la forme d'un bec d'enclenchement qui peut s'enclencher dans un logement d'enclenchement, de préférence périphérique, du connecteur enfichable (63).

3. Cartouche selon l'une quelconque des revendications précédentes, dans laquelle l'unité de raccordement présente un élément d'étanchéité (68).

4. Cartouche selon l'une quelconque des revendications précédentes, dans laquelle une partie (69) du tuyau (61), adjacente au connecteur enfichable (63), est coudée par rapport à l'axe longitudinal (66) et forme en particulier un angle de 90° avec l'axe longitudinal (66).

5. Système de chirurgie ophtalmologique (100) destiné au traitement d'un œil (6), comprenant au moins :
- une cartouche (4) qui présente au moins une pompe à fluide (10, 20, 30, 40) pour débiter un fluide de traitement,
- une console (1) qui présente la zone de réception de cartouche (56) pour recevoir la cartouche (4) et des moyens d'entraînement pour entraîner la pompe à fluide (10, 20, 30, 40) disposée dans la cartouche, et
- une pièce à main de chirurgie ophtalmologique (5) pour traiter un cristallin (7) de l'œil (6) et qui peut être accouplée de manière fluidique au moins à la cartouche (4),
dans lequel la cartouche (4) est réalisée selon l'une quelconque des revendications précédentes.
